Europäisches Patentamt

European Patent Office

Office européen des brevets

(19)

(11) Publication number: **0 117 705**
A2

# EUROPEAN PATENT APPLICATION

(12)

(21) Application number: **84301102.4**

(22) Date of filing: **21.02.84**

(51) Int. Cl.³: **A 61 K 39/395, C 12 N 5/00**

(30) Priority: **24.02.83 US 469608**

(43) Date of publication of application: **05.09.84**
**Bulletin 84/36**

(84) Designated Contracting States: **AT BE CH DE FR GB IT LI LU NL SE**

(71) Applicant: **THE REGENTS OF THE UNIVERSITY OF CALIFORNIA, 2199 Addison Street, Berkeley California 94720 (US)**

(72) Inventor: **Billing, Ronald, 5935 Lake Lindero, Agoura California (US)**
Inventor: **Terasaki, Paul I., 458 S. Bundy Drive, Los Angeles California (US)**

(74) Representative: **Harrison, David Christopher et al, MEWBURN ELLIS & CO 2/3 Cursitor Street, London EC4A 1BQ (GB)**

(54) **Monoclonal antibody specific for monocytes and blast cells.**

(57) Novel hybridomas can produce monoclonal antibodies capable of reacting with normal blood monocytes and blast cells. The antibodies are particularly useful as immunosuppressive agents which display few adverse side effects.

EP 0 117 705 A2

1

## MONOCLONAL ANTIBODY SPECIFIC
## FOR MONOCYTES AND BLAST CELLS

Monoclonal antibodies provide a sensitive tool for analyzing and detecting the presence of specific determinant sites. Many determinant sites of interest are found on highly complex structures, such as cells, where there are a seemingly limitless diversity of determinant sites. Frequently one is interested in being able to distinguish a particular type or group of cells which share common determinant site(s) for purposes of identification, separation or growth inhibition.

One particular area where monoclonal antibodies may find widespread application is in immunosuppression of patients undergoing skin grafts and other transplant procedures, as well as in treatment of patients having certain immune system disorders. While certain monoclonal antibodies specific for particular cell types have been used as immunosuppressants, the desired immunosuppression is often accompanied by adverse side effects. For example, treatment with OKT3 monoclonal antibody although generally effective, results in virtual depletion of normal blood lymphocytes leaving the patient susceptible to infection. Cosimi, et al. (1981) New Engl. J. Med. 305:308-314. It is thus desirable to provide monoclonal antibodies which afford immunosuppression without such adverse side effects.

Unfortunately, despite the seeming simplicity of the above stated goal, the preparation of hybridomas capable of producing antibodies having a desired specificity is problematic. The mixture of hybridomas which

2

result from a fusion often contain few, if any, hybridomas producing antibodies having high specificity for a determinant site of interest. The problem is intensified when it is desired to produce monoclonal antibodies specific for two or more cell types where it is unknown if the cell types have common determinant sites.

Cosimi, et al. (1981) supra. described the use of monoclonal antibodies specific for certain T cell subsets for immunosuppression. Several anti-blast hybridoma cell lines have been developed. See, e.g., Omary, et al. (1980) Nature 286 888-891 (B3-25 cell line); Haynes, et al. (1981) J. Immunol. 127:347-351 (5E9 cell line); and Reinherz, et al. (1980) PNAS 77:1588-1592 (OKT9 cell line). A polyclonal rabbit antiserum designated 157 which is specific for blast cells and peripheral blood monocytes has been developed. Billing, et al. (1979) Clin. Immunol. Immunopathol. 13:435-443.

According to the invention, monoclonal antibodies reactive with both monocytes and blast cells, the hybridomas which produce such monoclonal antibodies, and use of the monoclonal antibodies as immunosuppressants are provided.

More specifically, monoclonal antibodies which are specific for a determinant site common to both blast cells and monocytes are particularly useful in immunosuppression therapy for patients undergoing skin graft or other transplantation procedures. The subject monoclonal antibodies can also be used to identify and isolate the determinant site. The isolated determinant can then be used for the production of monoclonal antibodies specific to the same or substantially the same site as

3

the subject monoclonal antibodies but which may differ in class.

As used herein, the term blast cells includes those rapidly-dividing, partially-differentiated lymphoblastoid cells which are produced in response to antigenic challenge and which mature into B and T lymphocytes. The term monocyte includes all mononuclear phagocytes, specifically embracing macrophages which derive from monocytes.

Preferably, the monoclonal antibodies of the present invention will lack specificity for normal peripheral blood cells other than monocytes. In particular, the subject antibody will not react with normal peripheral T lymphocytes, B lymphocytes and granulocytes. In this way, a patient treated for immunosuppression with the subject antibody will retain the existing blood population of T and B lymphocytes to fight bacterial, viral and fungal infection. The production of new B and T lymphocytes, however, is inhibited through the suppression of blast cells and monocytes, resulting in increased patient tolerance to the transplant or graft.

The subject hybridomas and monoclonal antibodies are raised against a determinant site common to both blast cells and monocytes. Conveniently, blast cells or monocytes may be employed, either directly or as a source of the isolated determinant molecule.

In particular, the antibodies may be raised against a cultured T acute lymphocytic leukemia (T ALL) cell line designated CEM. One monoclonal antibody raised against the CEM cell line designated CBL1, which has been deposited at the ATCC on 24 February 1983 under acession No. HB8214, displayed cytotoxicity against a broad range of malignant cells, as well as against normal monocytes and blast cells. CBL1 displayed no reactivity with normal peripheral blood T lymphocytes, B lymphocytes, or granulocytes. Ascites fluid containing the monoclonal antibody was successfully used to prolong skin allograft

4

survival in Rhesus monkeys without apparent side effects.

The subject hybridomas and monoclonal antibodies can be obtained substantially following the well-known procedure of Kohler and Milstein, Eur. J. Immunol. (1976) 6:511-591. The method involves hyperimmunization of a mammalian host, e.g., a mouse, with the CEM cell line. Shortly after the last immunization, the host spleen is removed and the spleen cells fused with myeloma cells to produce hybridomas having a range of specificities. The hybridomas are selected on HAT media, and the supernatants screened for antibodies having the desired specificity. Those colonies which produce the desired antibodies may then be cloned by limiting dilution and grown out in flasks. Production of the desired antibodies may be enhanced by intraperitoneal injection of the hybridoma into a mammalian host to produce high-titered ascites fluid. In contrast to antisera derived directly from an immunized host, the monoclonal antibodies of the present invention are substantially free from immunoglobulins having differing specificities.

The antibodies of the subject invention can be used to isolate the common determinant site. This can be readily achieved by a number of conventional techniques. For example, the antibody may be used to prepare an immunosorbent column. By lysing the CEM cells, separating the cellular debris, and then passing the supernatant through the column, the molecules displaying the determinant will bind to the column. After washing the column to remove nonspecifically bound proteins, the antigen or determinant may be eluted, using detergents, urea, etc. in programmed gradient elution. The antigen may then be isolated and used to hyperimmunize an appropriate host followed by fusion of splenocytes with a fusion partner and screening for the desired hybridoma(s). In this way, monoclonal antibodies having substantially identical specificities, but which differ in class, such as IgM, IgG, IgA, IgE, and IgD, may be obtained. In addition, antibodies from a variety of different hosts, e.g., human, can be obtained.

The subject antibodies are particularly useful for immunosuppressive treatment. For such use, it is desirable to employ antibodies of class IgM, IgG2a, IgG2b or IgG3 which activate the host's own complement system to reduce the population of blast cells and monocytes. Alternatively, antibodies of any class may be coupled to cytotoxic agents to provide a composition lethal to blast cells and monocytes, but relatively nonlethal to the remainder of the normal peripheral blood population. Suitable cytotoxic agents include chlorambucil (Ghose et al., (1972), Cancer 29, 1398-1400; and Ghose et al. (1972) Br. Med. J. 3:495-499), $^{131}$I (Ghose et al. (1975), Cancer 36:1646-1657), whole diphtheria toxin (Moolten et al. (1972) J. Natl. Cancer Inst. 49:1059-1062; Moolten et al. ibid 55:473-477; and Thorpe et al. (1978) Nature 271:752-755), and the A chain of ricin toxin (Oeltmann et al. (1979) J. Biol. Chem. 254:1022-1027; and Oeltmann et al., ibid, 254:1028-1032). For administration to a patient, the antibodies will normally be combined with a physiologically-acceptable media, such as saline, phosphate buffered saline, serum, water, or other convenient carrier. The antibodies can be administered in a variety of ways, conveniently intravenously.

The subject antibodies will also find use in screening assays, particularly for the immunological classification of leukemia. Lymphoid leukemias can be divided into immunological subsets depending on the particular surface antigens displayed by the malignant cells. For example, CBL1 antibody is reactive with three out of the four generally recognized morphological types of leukemia, being nonreactive with chronic lymphocytic leukemia (CLL). CBL1 would thus be useful,

6

in combination with other antibodies having different specificities, in clinical diagnosis of a patient having leukemia. Malignant lymphocyte cells which are incapable of reacting with CBL1 would be presumed to be CLL.

The following experimental results are offered by way of example and not by way of limitation.


EXPERIMENTAL

The following abbreviations are employed: ALL-- acute lymphocytic leukemia; AML--acute myelomic leukemia; AMML--acute myelomonocytic leukemia; CML--chronic myelocytic leukemia; CLL--chronic lymphocytic leukemia; PBS-- phosphate buffered saline; PAGE--polyacrylamide gel electrophoresis; PHA--phytohemagglutinin; RBC--red blood cell; SDS--sodium dodecyl sulfate; and WBC--white blood cell.

MATERIAL AND METHODS

Cells

Leukemia Cells: Heparinized peripheral blood samples were drawn from children and adults with active leukemia. The acute lymphocytic leukemia (ALL) and acute myelocytic leukemia (AML) patients had peripheral blood blast counts greater than 90%. Leukemia cells from patients and peripheral blood lymphocytes from healthy donors were isolated by Ficoll-Hypaque[®] density gradient centrifugation. Leukemia cells were stored in liquid nitrogen.

Cell lines were grown in suspension cultures in RPMI 1640 containing 10% heat-inactivated fetal calf serum.

T and B Lymphocytes: T and B lymphocytes were prepared from whole lymphocytes by the nylon-wool method. Danilovs, et al. (1980) Histocompatibility Testing 1980, Terasaki (ed.) UCLA Tissue Typing Laboratory, Los Angeles, CA., pp. 287-288.

PHA Blasts: Whole peripheral blood lymphocytes isolated by the Ficoll-Hypaque[®] technique were cultured

at $2.5 \times 10^6$/ml in media 199 (M199) with 20% human AB serum (heat inactivated) at 37°C with 50µg/ml of Difco PHA under sterile conditions for 3-6 days. Control lymphocytes were incubated in M199 with 20% human AB serum without PHA. At the end of the culture period, cells were removed, washed, and tested by microcytotoxicity against the blast sera.

Monocytes: Monocytes were isolated using a Percoll® density gradient. Gutierrez, et al. (1979) Immunol. Meth. 29:57-63. Briefly, the thrombin pellet from lymphocyte isolation containing monocytes, platelets, and granulocytes was washed and resuspended in 65% Percoll® in PBS (by volume). Then 55% Percoll®, 40% Percoll®, and McCoy's media were layered, respectively, over the suspension and centrifuged at 3200 x g for 10 min with the brake off. The monocytes were recovered from the 40-55% Percoll® interface, diluted with media and washed.

Granulocytes: Granulocytes were isolated from Ficoll® pellets by removing RBCs by agglutination. The Ficoll® pellet from lymphocyte isolation containing granulocytes and RBCs was suspended in McCoy's media and centrifuged at 3000 x g for 1 min and the buffy coat from the pellet removed. This process was repeated until the buffy coat was sufficiently enriched for granulocytes. The appropriate agglutinin (anti-A, -B, -AB, -O, and -H) was added and allowed to agglutinate fully. The clumps were then spun at 1500 x g for less than 1 sec and the supernatant layered over Ficoll® (1.3545g/ml) and centrifuged at 3000 x g for 2 min. The granulocyte-enriched pellet was then washed and tested against antiblast sera. (This entire process was done in Fisher tubes using a Fisher, Model 59, centrifuge.)

Cytotoxicity

A microcytotoxicity test was performed by adding 1µl of antiserum at various dilutions to 1µl of cell suspension (2000 cells) in a microtest plate. The mixture was incubated at 22°C for 30 min, 5μl of rabbit complement was added, and incubation was continued for an additional 60 min. The reaction was then stained with eosin dye, fixed, and read for cytotoxicity. See, Billing, et al. (1979) Immunol. Immunopathol. 13:435-443.

Antibodies

Heteroantibody (designated heteroantibody 157) to cell line CEM (a lymphoblastoid T ALL cell line) was raised in rabbits immunized with CEM cells. Billings et al. (1979), supra.

Monoclonal antibodies against the CEM cell line were raised as follows. Two 6-week-old female Balb/c mice were immunized intravenously with $1-2 \times 10^7$ CEM cells weekly for three weeks. Three days after the final injection the spleens were removed and a single cell suspension was made. Spleen cells were fused with P3X63Ag8 myeloma cells at a ratio of 3:1 with 50% polyethylene glycol. After the fusion, hybrid cells were selected by hypoxanthine/aminopterin/thymidine treatment. After three weeks, supernatants were screened by microcytotoxicity against CEM cells. Several wells produced discriminating antibodies. One hybridoma, CBL1 which showed specificity for blast cells, was cloned and grown out in tissue culture flasks and as ascites in Balb/c mice.

Monoclonal antibodies against Ia positive leukemia cells (Reh cell line) were raised as follows. Female Balb/c mice (six weeks old) were immunized intravenously with $2 \times 10^7$ Ia positive leukemia cells (Reh) at 14-day intervals. Four days after the third immunization the spleens were removed and a suspension of single cells was prepared. Cell fusions were carried out by fusing with mouse myeloma cells (X63) according to the procedure developed by Kohler and Milstein (1976) Eur. J. Immunol. 6:511-519. After cell fusion the cells were distributed and cultured in selective medium. One hybrid clone produced a cytotoxic monoclonal antibody (H4) that reacted with B cells and other Ia positive cells but not T cells. It was an IgG3 antibody that bound to protein A. By immunoprecipitation and gel electrophoresis, H4 reacted with the human Ia-like antigen (p.27,35). Hocking et al. (1981) Blood 58:1040-1042.

Immunoprecipitation and Polyacrylamide
Gel Electrophoresis

Cells ($5 \times 10^6$ per experiment) were labeled with $^{125}$I by the iodogen technique of Markwell and Fox (1978) Biochemistry 17:4807-4817, solubilized with 300ml 0.5% Nonidet®P40, and immunoprecipitated with 20µl of antiserum 157 bound to 40µl protein A Sepharose®4B beads (Pharmacia) or 10µl CBL1 bound to 40µl rabbit antimouse IgM-protein A Sepharose®4B. After washing three times with PBS, the precipitated antigens were released from the protein A by adding 50µl 2% SDS. They were boiled for 2 min with or without dithiothreitol and run on 5% and 7% gels. Fifty 2mm slices from each gel were counted on a gamma counter. Standard proteins of known molecular weight were also run in order to calculate the molecular weight of the precipitated antigens. The gel system used was that originally described by King and Laemmli (1971) J. Mol. Biol. 62:467-480, and used by Billing et al. (1978) J. Natl. Cancer Inst. 61:423-429 The molecular weight and protein standards used were lysozyme 14,400, soybean trypsin inhibitor 21,500, carbonic anhydrase 31,000, ovalbumin 45,000, bovine serum albumin 68,000, phosphorylase B 92,900, and B-galactosidase 116,500.

Sequential immunoprecipitation experiments were performed using $^{125}$I-labeled lysate of CEM (T ALL line). CBL1 bound to rabbit antimouse IgM-protein A Sepharose®4B beads was then immunoprecipitated with antibody 157 bound to protein A Sepharose®4B.

Chromatofocusing

One milliliter of CBL1 ascites in 20ml 0.025M ethanolamine buffer was loaded on a 1x15cm column of Polybuffer Exchanger®94 (Pharmacia). Proteins were eluted from the column according to their isoelectric points with polybuffer 96 at pH 6.0. When the column reached pH 6.0, 1.0M NaCl was used as an eluent to remove all remaining proteins. Fractions were collected during the elution and the pH and $OD_{280nm}$ of each fraction was measured. In order to determine which fractions contained the antibody activity, each fraction was tested for complement-dependent cytotoxicity against blast cells and monocytes.

CFU-C test

This test, involving the growth of normal bone marrow colonies in soft agar with and without treatment with antibodies and complement is described in Billing et al. (1980) Clin. Immunol. Immunopathol. 16:202-210.

Animals

Young male or female Rhesus monkeys (Macaca mulatta) from the California Primate Research Center were used. Initially, two monkeys suffering from endometriosis were used because of the unknown possibility that fatal side-effects might occur. Later, healthy animals weighing between 5 and 6kg were used. Four animals were used as controls; 10 were treated with anti-Ia, and six with antiblast/monocyte antibodies.

Treatment of monkeys

The ascites form of antibodies was given daily by intravenous injection in 5ml saline. Control monkeys received similar injections of saline. The quantities of ascites given to each monkey are mentioned in the Results section. The initial dose was given two days prior to skin grafting. Then the same dose was given daily (except weekends) until the graft was rejected. In some cases the dose was increased after

11

the second day.  A full thickness 4cm x 4cm skin graft was applied on the forearm which was put in a plaster cast and removed on the fifth postoperative day. Discoloration of the graft was taken as the endpoint for graft rejection.

Monitoring

The total and differential counts of peripheral white blood cells were done daily.  The peripheral B cells were monitored daily be a rosetting technique using ox red blood cells coupled with the mouse H4 monoclonal antibody.  Rosetted cells were counted on a hemocytometer and expressed as a percentage of total lymphocytes.  Additionally, B cells were tested for indirect immunofluorescense using the H4 monoclonal antibody.  Using this technique it was possible to define the dosage and the timing of antibody administration required to provide a reduction in peripheral blood Ia positive cells in treated monkeys.  In the second experiment peripheral blood monocytes were counted daily.  Blood serum was also examined by immunodiffusion to detect antimouse antibodies in these primates.  None of the monkeys had detectable levels of these antibodies at any time during the administration of monoclonal antibody or following skin graft rejection.

RESULTS

The reactivity of monoclonal antibody CBL1 and heteroantiserum 157 was tested by cytotoxicity (Table 1) against a panel of normal and leukemia cells. The general reactivity pattern of the two antibodies was similar but not identical.  For example, both CBL1 and 157 reacted with leukemia blasts from the majority of AML, AMML, ALL, and CML in blast crisis.  Leukemia cells from 11 chronic lymphocytic leukemia (CLL) and 3 hairy cell leukemia patients were negative.  Eight of eight ALL cell lines and 6/6 PHA blast tested were positive to both CBL1 and 157.  However, apart from

12

monocytes, no other normal peripheral blood cells reacted with either CBL1 or 157.

TABLE 1

CYTOTOXICITY OF MONOCLONAL ANTIBODY CBL1
AND HETEROANTISERA 157 AGAINST A PANEL OF
NORMAL AND MALIGNANT CELLS

| Cell type | CBL1 No. pos./No. tested | 157 No. pos./No. tested |
|---|---|---|
| AML | 24/25 | 23/25 |
| ALL | 24/28 | 25/28 |
| CLL | 0/11 | 0/11 |
| CML Blast Crisis | 6/8 | 7/8 |
| Sezary | 3/6 | 3/6 |
| T ALL | 3/5 | 5/5 |
| AMML | 6/6 | 6/6 |
| Hairy Cell | 0/3 | 0/3 |
| Normal P.B. T Lymphocytes | 0/9 | 0/9 |
| Normal P.B. B Lymphocytes | 0/9 | 0/9 |
| Normal P.B. Granulocytes | 0/12 | 0/12 |
| Normal P.B. Monocytes | 8/8 | 8/8 |
| Platelets | 0/2 | 0/2 |
| PHA Blasts | 6/6 | 6/6 |
| Cell Lines[a] | 8/8 | 8/8 |

[a] Reh, CEM, 8402, HSB2 Conception (B lymphoma), Daudi, HL60, K562.

There were some definite differences in reactivity of CBL1 and 157 against certain leukemia cells which remained consistent after several repeated testings (Table 2).

In Table 2, AML cell 206371 is a representative cell which was clearly positive to both CBL1 and 157. Against this target cell the titer of CBL1 was $1:10^5$ and the titer of 157 was 1:128. The other six cells listed in Table 2 showed discrepancies between CBL1 and 157. For example, human blast cells from five patients reacted with 157 but not with CBL1 and one AML cell, 223836, reacted with CBL1 but not with 157.

13

## TABLE 2

### DIFFERENCES IN CYTOTOXICITY OF CBL1 COMPARED WITH 157 AGAINST CERTAIN LEUKEMIA CELLS

| Cell Type | I.D. | Medium control | CBL1 | | | | | | 157 | | | | | |
|---|---|---|---|---|---|---|---|---|---|---|---|---|---|---|
| | | | $10^1$ | $10^2$ | $10^3$ | $10^4$ | $10^5$ | $10^6$ | 4 | 8 | 16 | 32 | 64 | 128 |
| AML | 206371 | 1 | 8[a] | 8 | 8 | 8 | 6 | 1 | 8 | 8 | 8 | 8 | 8 | 8 |
| AML | 223836 | 1 | 8 | 8 | 8 | 6 | 6 | 1 | 4 | 1 | 1 | 1 | 1 | 1 |
| AML | 201192 | 1 | 8 | 1 | 1 | 1 | 1 | 1 | 8 | 8 | 8 | 8 | 2 | 1 |
| T ALL | 220308 | 1 | 1 | 1 | 1 | 1 | 1 | 1 | 8 | 8 | 8 | 1 | 1 | 1 |
| T ALL | 224702 | 1 | 6 | 1 | 1 | 1 | 1 | 1 | 8 | 8 | 8 | 8 | 1 | 1 |
| CML | 224270 | 1 | 1 | 1 | 1 | 1 | 1 | 1 | 8 | 8 | 8 | 8 | 6 | 6 |
| B lymphoma | 236232 | 1 | 4 | 4 | 1 | 1 | 1 | 1 | 8 | 8 | 8 | 8 | 8 | 8 |

[a]8, 80-100% dead cells; 6, 50-79%; 4,20-49%; 2, 10-19%, 1, 0-9%

CBL1 was strongly cytotoxic to myeloid colonies of normal bone-marrow cells grown in soft agar (Table 3). When bone-marrow cells were pretreated with complement and CBL1 at dilutions of $10^{-2}$, $10^{-3}$, and $10^{-4}$ there was a significant reduction in the number of CFU-C colonies.

## TABLE 3

### CYTOTOXICITY OF MONOCLONAL ANTIBODY CBL1 AGAINST CFU-C FROM NORMAL HUMAN BONE MARROW

| Antibody | Dilution | % Recovery CFU-C |
|---|---|---|
| -- | -- | 100 |
| CBL1 | 1:100 | 0 |
| CBL1 | 1:1000 | 0 |
| CBL1 | 1:10,000 | 44 |

CBL1 was tested against isolated cells from solid tumors. Apart from a lung and papillary carcinoma, 22 different solid tumor cells were negative. These included cells from four breast, five lung, four colon, four rectum, two stomach, and three ovarian tumors.

CBL1 ascites fluid was fractionated by chromatofocusing. One major cytotoxicity peak was eluted at a pH of 9.95 suggesting that the ascites contained only one cytotoxic antibody against the immunizing cell (CEM). This antibody reacted with blast cells and monocytes and was identified as IgM by immunodiffusion. Fraction 26, which contained only 1% of the total cytotoxic reactivity, contained several subclasses of IgG, IgM, and IgA. The proteins in fraction 26 probably represent IgG1 from the X63 myeloma genes and also normal mouse proteins that are part of all myeloma ascites.

The apparent molecular weights of the blast antigens detected by 157 and CBL1 were determined by immunoprecipitation of $^{125}$I-labeled cell surface antigens and SDS-PAGE. Antiserum 157 immunoprecipitated an antigen of 180,000 daltons under nonreducing conditions that dissociated into a single polypeptide of 90,000 under reducing conditions.

The antigen detected by CBL1 could not be determined by $^{125}$I-labeling and immunoprecipitation using the immunizing cell CEM (T ALL line). When labeled cell surface antigens from CEM were immunoprecipitated with CBL1 and run on SDS-PAGE, no labeled antigenic peaks were observed. Sequential immunoprecipitation experiments with 157 and CBL1 were done. $^{125}$I-labeled lysate from CEM was cleared with CBL1 bound to Sepharose beads. Then the supernatant remaining was immunoprecipitated with antibody 157 bound to Sepharose beads. SDS-PAGE of the bound antigen still showed 180,000 dalton peaks present after clearing the lysate with CBL1. This strongly indicated that the CBL1 antibody was not removing, and thus not reacting with the 180,000 dalton antigen recognized by antiserum 157.

15

## Treatment with CBL1 as Immunosuppressant

The cytotoxicity of CBL1 against normal cells was presented in Table 1. All monocytes and blast cells were positive (titer $1:10^{-4}$) while normal nondividing cells were negative.

Six Rhesus monkeys treated with CBL1 ascites received skin transplants which survived from 16 to 22 days (Table 4). No deaths or adverse side effects were seen following treatment with CBL1. Differential blood cell counts taken every other day did not show any significant changes in lymphocytes, granulocytes, platelets, or monocytes.

### TABLE 4

SKIN GRAFT SURVIVAL IN MONKEYS TREATED WITH MONOCLONAL ANTIBLAST/MONOCYTE ANTIBODY.

| Exp.# | Animal# | Titer | Dose/Lamda | Skin graft survival |
|-------|---------|-------|------------|---------------------|
| 1. | 17182 | $10^4$ | 300 | 16 days |
| 2. | 17083 | $10^4$ | 300 | 16 days |
| 3. | 17085 | $10^4$ | 300 | 15 days |
| 4. | 7633 | $10^4$ | 450 | 22 days |
| 5. | 7212 | $10^4$ | 450 | 20 days |
| 6. | 7253 | $10^4$ | 450 | 20 days |

## Treatment with H4 as Immunosuppressant

Table 5 shows the complement dependent cytotoxicity of the ascites form of anti-Ia monoclonal antibody (H4) against a panel of normal cells. The titer was between $1:10^4$ and $1:10^5$ against B cells, monocytes, and spleen cells. T cells were unreactive.

16

The H4 antibodies were also shown by immunoprecipitation to react with the Ia antigen p27,35. Hocking et al. (1981), <u>supra</u>.

TABLE 5

COMPLEMENT-DEPENDENT CYTOTOXICITY
OF ANTI-IA MONOCLONAL ANTIBODY (H4)

| Cell Tested | No. of samples positive/no.tested | Reciprocal titers |
|---|---|---|
| Peripheral blood T lymphocytes | 0/100 | -- |
| Normal spleen cells | 40/40 | $10^4-10^5$ |
| Normal monocytes | 15/15 | $10^4-10^5$ |
| Normal granulocytes | 0/16 | -- |
| Normal platelets | 0/4 | -- |
| T-cell lines | 0/5 | -- |
| B-cell lines | 5/5 | -- |

The use of H4 which is reactive with blast cells and monocytes, but which further displays reactivity with mature B lymphocytes and Ia antigen, was also examined.

Results of the skin-graft experiments employing H4 are summarized in Table 6. Three monkeys died before a safe dosage of H4 was determined.

TABLE 6

SKIN-GRAFT SURVIVAL IN RHESUS MONKEYS
TREATED WITH MONOCLONAL ANTI-IA ANTIBODIES (H4)

| Exp.# | Animal# | Titer | Dose Lambda | Survival of skin graft | Comments |
|-------|---------|-------|-------------|------------------------|----------|
| 1. | 7895 | $10^5$ | 1000 | N.A. | Died |
| 2. | 8048 | $10^5$ | 500 | N.A. | Died |
| 3. | 7898 | $10^5$ | 250 | N.A. | Died |
| 4. | 7565 | $10^5$ | 20-50 | 19 days | Alive and well |
| 5. | 7681 | $10^4$ | 100-500 | * | Animal chewed the graft through the cast in the upper arm on the 5th day |
| 6. | 7531 | $10^4$ | 500 | 14 days | Alive and well |
| 7. | 7862 | $10^5$ | 50-75 | 15 days | Alive and well |
| 8. | 7484 | $10^4$ | 100-250 | 13 days | Alive and well |
| 9. | 6596 | $10^5$ | 50-125 | 15 days | Alive and well |
| 10. | 6587 | $10^5$ | 100-500 | * | Died on 6th day with viable graft |

| | | CONTROL MONKEYS | | | |
|-------|---------|-------|-------------|------------------------|----------|
| 1. | 7633 | | Nil | 5 days | Alive and well |
| 2. | 17083 | | Nil | 5 days | Alive and well |
| 3. | 17085 | | Nil | 5 days | Alive and well |
| 4. | 17182 | | Nil | 6 days | Alive and well |

Since no prior knowledge existed in the literature with regard to what would be considered a "safe" dose of anti-Ia, the first three monkeys received 1.0ml, 0.5ml, and 0.25ml of a batch of H4 with a titer of 1:$10^5$ in 5ml saline. All these doses proved to be fatal and the animals died within hours of receiving the antibodies. The cause of death appeared to be directly related to the cytotoxic effect of the H4 ascites fluid, because a similar volume of ascitic fluid with a lower cytotoxic titer did not have any adverse effects on the primates. Thereafter there was

18

only one death that occurred on the sixth postoperative day which was again attributable to administration of a higher dose of H4. The animals appeared to die from an anaphylactic type reaction, apparently resulting from the Ia-specific reaction with mast cells which can release toxic levels of histamines.

Meaningful skin-transplant results were obtained from five monkeys treated with anti-Ia. All of these were alive and well during the treatment. The four control animals rejected their grafts on the fifth or sixth days whereas the five anti-Ia animals rejected between 13 and 19 days (Table 6).

The number and percentage of B lymphocytes in the peripheral blood of the Ia-treated monkeys was reduced during treatment but invariably began to increase again towards the rejection time. Table 7 shows the blood counts of one representative monkey (#7862). Similar results were obtained with the other anti-Ia treated monkeys. The number of B lymphocytes decreased from $1,011 \times 10^3/mm^3$ before treatment to between 173 and $250 \times 10^3/mm^3$ during treatment but began to increase to $330 \times 10^3/mm^3$ at rejection. Total WBC and platelet counts remained fairly constant during treatment.

19

TABLE 7

REPRESENTATIVE BLOOD CELL COUNTS
IN AN ANIMAL TREATED WITH
ANTI-IA ANTIBODY (H4)

|  | Pretreatment with H4 | Days following skin graft | | | | |
|---|---|---|---|---|---|---|
|  |  | Day 1 | Day 4 | Day 7 | Day 12 | Day 15 |
| WBC($10^3$/mm$^3$) | 7.2 | 8.1 | 5.4 | 6.0 | 5.7 | 5.8 |
| Lympho % | 52% | 22% | 31% | 17% | 40% | 38% |
| Platelets/mm$^3$ | 429,000 | -- | 439,000 | 331,000 | 535,000 | -- |
| B-cells (% of lymphocytes) | 27% | 13% | 11.8% | 17% | 11% | 14% |
| Absolute count of B-cells/mm$^3$ | 1101 | 231 | 197 | 173 | 250 | 330 |
|  |  |  |  |  |  | (Graft rejected) |

It is evident from the above results that the
subject monoclonal antibodies are specific for a
particular subpopulation of leukocytes including blast
cells and monocytes. The antibodies may be used as
immunosuppressive agents which prolong survival of
transplants. Moreover, the antibodies promise fewer
side effects than pancytotoxic drugs which have a broad
spectrum suppressive effect on the immune system. In
particular, by leaving the existing population of
mature B cells and T cells intact to fight bacterial,
viral, and fungal infections, the patient's ability to
survive is greatly enhanced. The use of monoclonal
antibodies which further react with Ia antigen, as
typified by H4, is contraindicated since such reactivity
can be lethal.

20

Although the foregoing invention has been described in some detail by way of illustration and example for purposes of clarity of understanding, it will be obvious that certain changes and modifications may be practiced.

CLAIMS:-

1.      A composition comprising monoclonal antibodies specific for blast cells and monocytes but which substantially lack affinity for normal peripheral blood leukocytes other than monocytes, said composition being substantially free from other antibodies.

2.      A composition as claimed in Claim 1, comprising murine monoclonal antibodies.

3.      A composition as claimed in Claim 1 or Claim 2, comprising monoclonal antibodies selected from the group consisting of IgM, IgG2a, IgG2b, and IgG3.

4.      A composition as claimed in Claims 1 to 3, comprising monoclonal antibodies which are substantially free from Ia specificity.

5.      A composition as in any one of the preceding Claims, wherein said monoclonal antibodies are coupled to a cytotoxic agent.

6.      Hybridoma cells producing monoclonal antibodies specific for blast cells and monocytes but which substantially lack affinity for normal peripheral blood leukocytes other than monocytes, said composition being substantially free from other antibodies.

7.      Cells as claimed in Claim 6 which are murine hybridoma cells.

8.      Hybridoma cells as claimed in Claim 6 or Claim 7 which produce antibodies selected from the group consisting of IgM, IgG2a, IgG2b, and IgG3.

9.      Hybridoma cells as claimed in Claim 6, Claim 7

or Claim 8 which produce antibodies which are substantially free from Ia specificity.

10. A murine hybridoma cell line having ATCC accession No. HB 8214.

11. Antibodies produced by said hybridoma cell line or Claim 10 or which bind with substantially the same affinity to the same epitopic site as antibodies produced by the said hybridoma cell line.

12. An immunosuppressive preparation comprising antibodies according to Claim 10 or Claim 11 or a composite according to any one of Claims 1 to 5.